# FASCICULE DE BREVET EUROPEEN

(11) **EP 2 916 871 B1**
(45) Date de publication et mention de la délivrance du brevet: **14.09.2016**
(21) Numéro de dépôt: 13801639.9
(22) Date de dépôt: 07.11.2013
(51) Int. Cl.: A61K 47/20, A61K 9/14, A61K 31/57, A61P 15/00, A61P 15/18, A61K 9/16

(54) **PRODUIT DE CO-MICRONISATION COMPRENANT DE L'ULIPRISTAL ACETATE**
CO-MIKRONISATIONSPRODUKT ENTHALTEND ULIPRISTAL ACETAT
CO-MICRONISATION PRODUCT COMPRISING ULIPRISTAL ACETATE

(30) Priorité: 08.11.2012 FR 1260603
(43) Date de publication de la demande: 16.09.2015
(73) Titulaire: Laboratoire HRA Pharma, 75003 Paris (FR)
(72) Inventeur: BATTUNG, Florian, F-75013 Paris (FR); JUVIN, Pierre-Yves, F-44100 Nantes (FR); HECQ, Jérôme, F-33360 Camblanes et Meynac (FR); COLIN, Aude, F-33100 Bordeaux (FR)
(74) Mandataire: Cabinet Becker et Associés
(86) Numéro de dépôt international: PCT/FR2013/052670
(87) Numéro de publication internationale: WO 2014/072646

(56) Documents cités:
- EP-A1- 2 471 537
- WO-A1-00/28970
- US-A1- 2009 192 130

## Description

### Domaine de l'invention

La présente invention concerne une nouvelle forme galénique de l'ulipristal acétate, plus précisément, un produit de co-micronisation, et des compositions pharmaceutiques contenant ladite forme galénique.

### Arrière-plan technologique de l'invention

L'ulipristal acétate (en abrégé UPA) correspond à la 17α-acétoxy-11β-[4-(N,N-diméthylamino)-phényl]-19-norprégna- 4, 9-diène-3,20-dione (nomenclature IUPAC) et possède la formule chimique suivante :

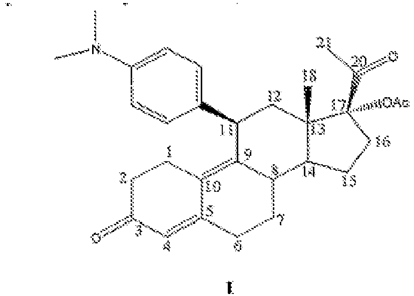

Sa synthèse est décrite, entre autres, dans le brevet EP 0 422 100 et dans la demande de brevet EP 1 602 662.

L'ulipristal acétate est un modulateur synthétique sélectif des récepteurs de la progestérone. Par son action sur le récepteur de la progestérone, l'ulipristal acétate est capable d'exercer une action contraceptive en inhibant ou en retardant de l'ovulation. Des études cliniques ont montré que l'ulipristal acétate, administré en une dose unique de 30 mg, permet d'éviter une grossesse non désirée quand il est administré dans les 120 heures suivant un rapport sexuel non ou mal protégé (Glasier et al, Lancet. 2010, 375(9714):555-62 ; Fine et al, Obstet Gynecol. 2010, 115:257-63). L'ulipristal acétate a été ainsi autorisé en tant que contraceptif d'urgence et est commercialisé sous le nom commercial EllaOne® en Europe.

D'autres applications thérapeutiques de l'ulipristal acétate ont été proposées dans l'état de la technique. Des essais cliniques récents ont montré que l'administration chronique d'ulipristal acétate (à raison de 5mg ou 10 mg par jour) permet de réduire de manière significative les symptômes associés aux fibromes utérins et fournit un bénéfice thérapeutique supérieur à celui du traitement de référence, à savoir, l'acétate de leuprolide (Donnez et al., N Engl J Med. 2012; 366(5):421-32.). Sur la base de ces essais cliniques, l'Agence Européenne du Médicament (EMEA) a autorisé, en février 2012, la spécialité Esmya ® (5mg d'ulipristal acétate) pour le traitement pré-opératoire des symptômes associés aux fibromes utérins.

Les compositions pharmaceutiques actuellement commercialisées comportent de l'ulipristal acétate sous une forme micronisée.

La spécialité Esmya® se présente sous la forme d'un comprimé non-pelliculé comprenant 5 mg d'ulipristal acétate micronisé associés aux excipients suivants : cellulose microcristalline, mannitol, croscarmellose sodique, talc et stéarate de magnésium.

EllaOne® se présente, quant à elle, sous la forme d'un comprimé non-pelliculé comprenant 30 mg d'ulipristal acétate micronisé et les excipients suivants : lactose monohydraté, povidone K30, croscarmellose sodique et stéarate de magnésium.

Des compositions pharmaceutiques supplémentaires ont été décrites dans la demande internationale WO 2010/066749.

La mise au point de nouvelles formes galéniques adaptées pour l'administration de l'ulipristal acétate demeure un enjeu majeur pour les utilisations thérapeutique et contraceptive de l'ulipristal acétate.

A cet égard, il existe, à l'heure actuelle, un besoin de nouvelles formulations pharmaceutiques contenant de l'ulipristal acétate et présentant des propriétés de libération et une biodisponibilité adaptées.

### Résumé de l'invention

La présente invention a pour objet un produit de co-micronisation comprenant (i) un principe actif choisi parmi le groupe constitué par l'ulipristal acétate, un métabolite de l'ulipristal acétate et leurs mélanges, et (ii) un surfactant solide acceptable sur le plan pharmaceutique.

Dans certains modes de réalisation, le produit de co-micronisation selon l'invention a une ou plusieurs des caractéristiques suivantes :
- le rapport massique entre le principe actif et le surfactant est compris dans une gamme allant de 0,1 à 10, de préférence 0,5 à 4,
- le surfactant est choisi parmi les sels d'alkylsulfates en C₈-C₂₀ et leurs mélanges, de préférence, le dodécylsulfate de sodium,
- le principe actif est choisi parmi le groupe constitué par l'ulipristal acétate, la 17α-acétoxy-11β-(4-N-méthylamino-phényl)-19-norprégna-4,9-diène-3, 20-dione, la 17a-acétoxy-11β-(4-amino-phényl)-19-norprégna- 4, 9-diène-3,20-dione et leurs mélanges, et/ou
- une d50 inférieure à 20 µm, de préférence inférieure à 15 µm, et/ou une d90 inférieure à 50 µm, de préférence inférieure à 40 µm.

La présente invention a également pour objet un procédé de préparation d'un produit de co-micronisation tel que défini précédemment, comprenant les étapes consistant à :
a) fournir un principe actif choisi parmi le groupe constitué par l'ulipristal acétate, un métabolite de l'ulipristal acétate et leurs mélanges,
b) mélanger le principe actif de l'étape a) avec le surfactant et
c) co-microniser le mélange obtenu à l'étape b).

Le principe actif de l'étape a) peut être sous forme micronisée ou non micronisée.

Un objet supplémentaire de l'invention est une composition pharmaceutique comprenant un produit de co-micronisation tel que défini précédemment et un excipient acceptable sur le plan pharmaceutique. L'excipient acceptable sur le plan pharmaceutique est, de préférence, choisi parmi le groupe constitué par un diluant, un liant, un agent d'écoulement, un lubrifiant, un agent désintégrant et leurs mélanges.

Dans certains modes de réalisation, la composition pharmaceutique comprend :
- 0,5% à 80% de produit de co-micronisation,
- 0% à 10% d'agent désintégrant,
- 15% à 95 % de diluant, et
- 0% à 5% de lubrifiant,
les pourcentages étant exprimés en poids par rapport au poids total de la composition.

De préférence, la composition pharmaceutique selon l'invention comprend de 1 mg à 100 mg de préférence de 1 mg à 40 mg de principe actif par unité de dose. Elle est de préférence destinée à une administration par voie orale et peut se présenter sous la forme d'une poudre, d'une granule, d'un comprimé pelliculé ou non pelliculé, ou d'une gélule.

La présente invention a également pour objet un produit de co-micronisation ou une composition pharmaceutique tels que définis précédemment pour une utilisation en tant que contraceptif par exemple, en tant que contraceptif régulier ou en tant que contraceptif d'urgence.

Enfin, l'invention a également pour objet un produit de co-micronisation ou une composition pharmaceutique, tels que définis précédemment, pour une utilisation dans le traitement ou la prévention d'un trouble gynécologique, de préférence affectant l'utérus.

### Figures

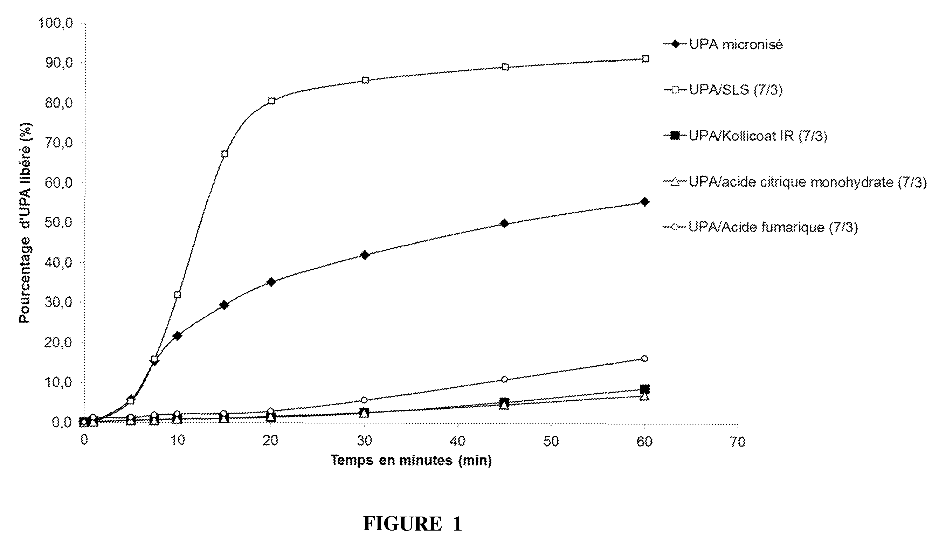

La **Figure 1** montre les courbes de dissolution *in vitro* pour différents co-micronisats (voir Exemple 1 ci-après) : UPA/SDS 7/3 (carré vide), UPA/kollicoat® IR 7/3 (carré plein), UPA/acide citrique monohydrate 7/3 (triangle vide), UPA/acide fumarique 7/3 (rond vide). Expérience témoin : UPA micronisé (seul - en absence d'excipient) (losange plein). Ordonnées : pourcentage d'UPA libéré (%), abscisse : temps en minutes.

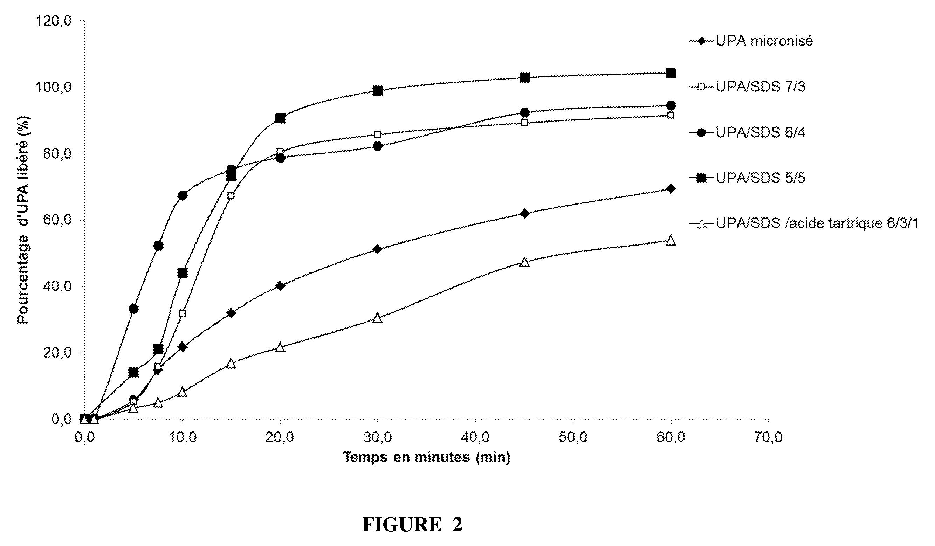

La **Figure 2** montre les courbes de dissolution *in vitro* pour différents co-micronisats (voir Exemple 1 ci-après) : UPA/SDS 7/3 (carré vide), UPA/SDS 6/4 (rond plein), UPA/SDS 5/5 (carré plein), UPA/SDS/acide tartrique 6/3/1 (triangle vide), Expérience témoin : UPA micronisé (seul - en absence d'excipient - (losange plein)). Ordonnées : pourcentage d'UPA libéré (%), abscisse : temps en minutes.

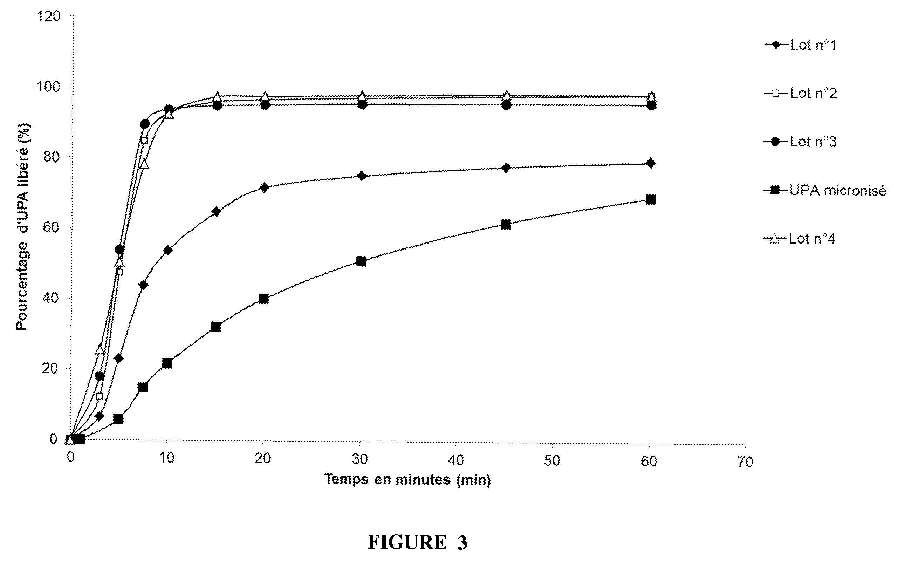

La **Figure 3** montre les courbes de dissolution *in vitro* pour différents lots de co-micronisats UPA/SDS 1/1 se différenciant par leur granulométrie et/ou la source d'UPA (voir Exemple 3 ci-après) : Lot n°1 (losange plein), lot n°2 (carré vide), lot n°3 (rond plein), lot n°4 (triangle vide), Expérience témoin : UPA micronisé (seul - en absence d'excipient - (carré plein)). Ordonnées : pourcentage d'UPA libéré (%), abscisse : temps en minutes.

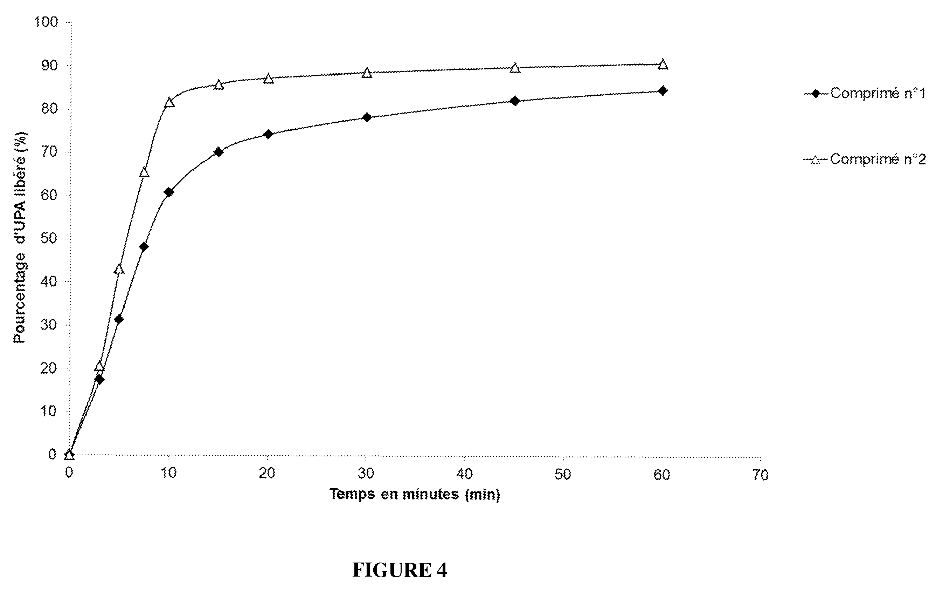

La **Figure 4** montre les courbes de dissolution *in vitro* pour des comprimés comprenant le co-micronisat UPA/SDS 1/1 (voir Exemple 6 ci-après) : Comprimé n°1 (losange plein), Comprimé n°2 (triangle vide), Ordonnées : pourcentage d'UPA libéré (%), abscisse : temps en minutes.

### Description détaillée de l'invention

### Produit de co-micronisation selon l'invention et procédé de préparation

A l'issue de longues recherches, le Demandeur a montré qu'il est possible d'améliorer de manière significative la dissolution *in vitro* et la biodisponibilité *in vivo* de l'ulipristal acétate (ci-après UPA) grâce à une technologie de co-micronisation.

De manière surprenante, le Demandeur a montré que le produit résultant de la co-micronisation de l'ulipristal acétate et du dodécylsulfate de sodium (également appelé ci-après SDS ou laurylsulfate de sodium) présentait une vitesse de dissolution *in vitro* significativement supérieure à celle de l'UPA micronisé seul, en l'absence d'excipient. Cette augmentation de la vitesse de dissolution de l'UPA est également observée lorsque le produit de co-micronisation est intégré dans une composition pharmaceutique (voir Exemple 6 ci-après). L'effet positif de la co-micronisation sur les propriétés de l'UPA a été confirmé *in vivo* par des études pharmacocinétiques conduites chez l'animal par le Demandeur. Ces études ont mis en évidence que le produit de co-micronisation UPA/SDS présente une biodisponibilité et une vitesse d'absorption pour l'UPA plus élevées que celles observées pour l'UPA sous forme micronisée (voir Exemple 5 ci-après).

Par ses propriétés pharmacocinétiques améliorées, il est attendu que le produit de co-micronisation permette de diminuer les doses d'UPA à administrer pour obtenir l'effet thérapeutique ou contraceptif recherché. La diminution de la dose d'UPA devrait permettre, entre autres, d'augmenter la sécurité, en particulier l'innocuité, des compositions pharmaceutiques finales. Le Demandeur a montré que la vitesse de dissolution et les propriétés pharmacocinétiques de l'UPA dans le produit de co-micronisation sont particulièrement améliorées lorsqu'on utilise un surfactant, en particulier le dodécylsulfate de sodium, en tant qu'excipient de co-micronisation. De manière remarquable, la co-micronisation ne conduit pas systématiquement à une amélioration des propriétés de dissolution de l'UPA. Les excipients de co-micronisation testés par le Demandeur dans l'exemple 1 ci-après, qui ne sont pas des surfactants, se sont révélés inefficaces pour améliorer les propriétés de dissolution *in vitro* de l'UPA.

En particulier, contrairement à ce que l'homme du métier aurait pu anticiper au regard des propriétés de dissolution de l'UPA en milieu acide, la co-micronisation de l'UPA avec un acide organique a conduit à une diminution nette de la vitesse de dissolution *in vitro* (voir Exemple 1, ci-après). Par ailleurs, le Demandeur a mis en évidence que le produit obtenu par mélange intime de SDS et d'UPA micronisé présente des propriétés de dissolution *in vitro* inférieures à celles du produit résultant de la co-micronisation dudit mélange (voir Exemple 2), même après incorporation dans une composition pharmaceutique (voir Exemple 6). L'ensemble de ces résultats souligne que l'amélioration des propriétés de dissolution de l'UPA dans les co-micronisats résulte de la combinaison spécifique (i) de la technologie de co-micronisation et (ii) de l'utilisation d'un surfactant, en particulier le SDS, en tant qu'excipient de co-micronisation.

Ainsi, la présente invention a pour objet une nouvelle forme galénique, plus précisément, un produit de co-micronisation comprenant :
- un principe actif choisi parmi le groupe constitué par l'ulipristal acétate, un métabolite de l'ulipristal acétate et leurs mélanges, et
- un surfactant solide acceptable sur le plan pharmaceutique.

Par produit de co-micronisation (également désigné ci-après co-micronisat), on entend le produit obtenu par micronisation d'un mélange comprenant un principe actif et au moins un excipient. Dans le cas d'espèce, il s'agit d'un mélange solide sous forme d'une poudre.

Dans le contexte de la présente invention, on entend par « micronisation » un procédé permettant de réduire la taille des particules d'une poudre, par exemple, par broyage. La réduction de la taille des particules est matérialisée par une diminution d'au moins 10% d'un paramètre choisi parmi la d50, la d10 et la d90. Une réduction « d'au moins 10% » englobe une réduction d'au moins 20%, d'au moins 30%, d'au moins 40%, d'au moins 50%.

La micronisation peut être effectuée au moyen de dispositifs disponibles dans le commerce, tels que les microniseurs à boulets ou à jet d'air.

Dans le contexte de la présente invention, on entend par « un produit micronisé » un produit se présentant sous la forme d'une poudre ayant une d90 inférieure à 50 µm. Ainsi, de manière préférée, le produit de co-micronisation selon l'invention a une d90 inférieure à 50 µm.

Des métabolites de l'UPA sont décrits, entre autres, dans Attardi et al., Journal of Steroid Biochemistry and Molecular Biology, 2004, 88 : 277-288 et illustrés ci-après:

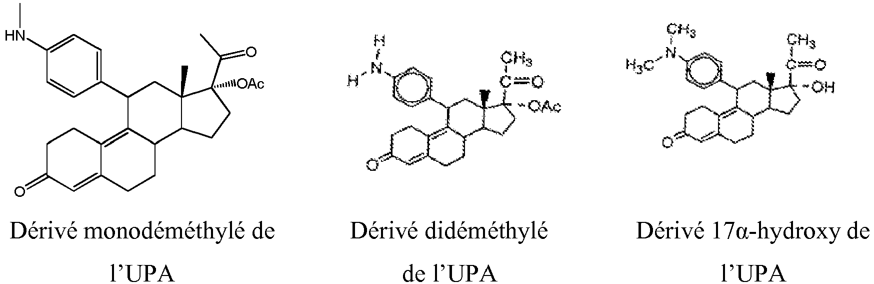

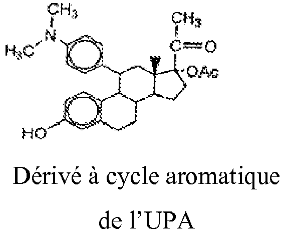

De préférence, le métabolite de l'ulipristal acétate est choisi parmi:
- la 17α-acétoxy-11β-(4-N-méthylamino-phényl)-19-norprégna-4,9-diène-3,20-dione (dérivé monodéméthylé) et
- la 17α-acétoxy-11β-(4-amino-phényl)-19-norprégna-4,9-diène-3,20-dione (dérivé didéméthylé).

Dans un mode de réalisation préféré du co-micronisat selon l'invention, le principe actif est sélectionné parmi le groupe constitué par la 17α-acétoxy-11β-[4-N-méthylamino-phényl)-19-norprégna-4,9-diène-3,20-dione, la 17α-acétoxy-11β-[4-amino-phényl)-19-norprégna-4,9-diène-3,20-dione, l'ulipristal acétate et leurs mélanges.

Le surfactant acceptable sur le plan pharmaceutique est choisi, de préférence, parmi les surfactants communément utilisés en galénique pouvant subir une co-micronisation et leurs mélanges. On entend par « surfactant solide » un surfactant qui est solide à température ambiante, c'est-à-dire typiquement à environ 20°C. Dans certains modes de réalisation avantageux, le surfactant présente une température de fusion élevée, de préférence supérieure à 50°C et de manière encore plus préférée supérieure à 100°C. De préférence, le surfactant est choisi parmi les sels d'alkylsulfates en C₈-C₂₀, de préférence en C₁₀-C₁₄ et leurs mélanges.

Dans un mode de réalisation avantageux, le surfactant est choisi parmi les sels de dodécylsulfate, de préférence, les sels alcalins ou alcalino-terreux de celui-ci tel qu'un sel de sodium, de magnésium ou de calcium.

Comme cela est démontré de manière exhaustive par les exemples de la présente demande, un surfactant particulièrement adapté pour l'obtention d'un produit de co-micronisation selon l'invention est le SDS c'est-à-dire le dodécylsulfate de sodium également appelé laurylsulfate de sodium (abréviation SLS en anglais). Ainsi, dans un mode de réalisation préféré, le surfactant est le dodécyl-sulfate de sodium.

Dans d'autres modes de réalisation, le produit de co-micronisation selon l'invention comprend :
- un principe actif choisi parmi le groupe constitué par parmi le groupe constitué par la 17α-acétoxy-11β-[4-N-méthylamino-phényl)-19-norprégna-4,9-diène-3,20-dione, 1a17α-acétoxy-11β-[4-amino-phényl)-19-norprégna-4,9-diène-3,20-dione, l'ulipristal acétate et leurs mélanges, de préférence l'ulipristal acétate, et
- le dodécylsulfate de sodium en tant qu'excipient de co-micronisation.

Le rapport massique entre le principe actif et le surfactant est généralement compris dans une gamme allant de 0,1 à 10, de préférence, de 0,5 à 4. Un rapport massique principe actif/surfactant de 0,5 à 4 englobe un rapport massique de 0,5 à 1, de 1 à 1,5, de 1,5 à 2, de 2 à 2,5, de 3 à 3,5 et de 3,5 à 4.

De préférence, le rapport massique principe actif/surfactant est compris dans une gamme allant de 0,8 à 2,5. Un rapport massique principe actif/surfactant adapté est, par exemple, un rapport massique allant de 0,8 à 1,2 tel qu'un rapport massique de 1. Comme cela a été montré dans les exemples, la granulométrie (c'est-à-dire la distribution de la taille des particules) du produit de co-micronisation peut avoir une incidence sur les propriétés de solubilité de l'UPA. Il est préférable que la d50 du produit de co-micronisation soit inférieure à 25 µm, de préférence inférieure à 20 µm, voire inférieure à 15 µm.

Une d50 inférieure à 15 µm englobe une d50 inférieure à 12 µm, à 11 µm, à 10 µm, à 9 µm, à 8 µm, à 7 µm, à 6 µm, à 5 µm, 4 µm.

Il est également préférable que la d90 du produit de co-micronisation soit inférieure à 50 µm, voire inférieure à 40 µm. Une d90 inférieure à 40 µm englobe une d90 inférieure à 38 µm, à 37 µm, à 36 µm, à 35 µm, à 34 µm, à 33 µm, à 32 µm, à 31 µm, à 30 µm, à 29 µm, à 28 µm, à 27 µm, à 26 µm, à 25 µm, à 24 µm, à 23 µm, à 22 µm, à 21 µm, à 20 µm, à 19 µm, à 18 µm, à 17 µm, à 16 µm, à 15 µm, à 14 µm, 13 µm, 12 µm, à 11 µm, à 10 µm.

Dans certains modes de réalisation, le produit de co-micronisation selon l'invention est caractérisé en ce que sa granulométrie présente :
- une d50 inférieure à 20 µm, de préférence inférieure à 15 µm, et/ou
- une d90 inférieure à 50 µm, de préférence inférieure à 40 µm, et de manière encore plus préférée inférieure à 30 µm,

A titre d'exemple, le co-micronisat selon l'invention peut avoir une d50 inférieure à 5µm et/ou une d90 inférieure à 15 µm.

La d10 du co-micronisat selon l'invention est généralement supérieure à 0,05 µm. Dans le contexte de la présente invention, « une d50 inférieure à X µm » signifie qu'au moins 50% des particules du co-micronisat ont une taille inférieure à X µm.
« une d90 inférieure à Y µm » signifie qu'au moins 90% des particules du co-micronisat ont une taille inférieure à Y µm.

De même, « une d10 supérieure à Z µm » signifie qu'au moins 90% des particules du co-micronisat ont une taille de particule supérieure à Z µm.

La granulométrie - c'est-à-dire la distribution de la taille des particules - du produit de co-micronisation - et en particulier les paramètres d90, d50 et d10 - peuvent être déterminés par toute méthode connue de l'homme du métier. De manière préférée, on utilisera la diffraction laser. L'exemple 3 ci-après propose des conditions de mise en oeuvre de cette méthode.

Dans certains modes de réalisation, le co-micronisat peut comprendre un ou plusieurs excipients supplémentaires au surfactant. Le ou les excipients supplémentaires peuvent être choisis parmi un diluant, un liant, un désintégrant et leurs mélanges. Dans certains modes de réalisation, le ou les excipients supplémentaires sont polymériques. A titre d'exemple, ils peuvent être choisis parmi les polymères et les copolymères de N-vinyl-2-pyrrolidone tels qu'une copovidone, une povidone ou une crospovidone.

Dans certains modes particuliers de réalisation, le co-micronisat selon l'invention comprend :
- un principe actif choisi parmi le groupe constitué par l'ulipristal acétate, un métabolite de l'ulipristal acétate et leurs mélanges,
- un surfactant solide acceptable sur le plan pharmaceutique, de préférence le SDS, et
- un excipient supplémentaire choisi parmi le groupe constitué par les polymères et les copolymères de N-vinyl-2-pyrrolidone et leurs mélanges, de préférence
une crospovidone, une povidone et leurs mélanges.

L'excipient supplémentaire peut être présent en une quantité correspondant à un rapport massique « principe actif / excipient supplémentaire » de 0,1 à 10, de préférence de 0,5 à 4.

Dans un mode de réalisation supplémentaire, le co-micronisat est dépourvu d'excipient supplémentaire, c'est-à-dire dépourvu d'un excipient autre que le surfactant. En particulier, le co-micronisat selon l'invention peut être constitué du principe actif et du surfactant.

Comme cela est illustré dans les exemples, le produit de co-micronisation présente des propriétés améliorées de biodisponibilité et de dissolution *in vitro* du principe actif. Dans certains modes de réalisation, le produit de co-micronisation selon l'invention est caractérisé en ce qu'au moins 80% du principe actif qu'il contient est libéré en 30 minutes lorsque ledit produit de co-micronisation est soumis à un test de dissolution *in vitro,* de préférence tel que défini dans la Pharmacopée Européenne §2.9.3.

Le test de dissolution *in vitro* peut être réalisé à l'aide de tout dispositif à pales disponible dans le commerce. L'exemple 1 ci-après présente des conditions de mise en oeuvre pour la détermination de la vitesse de dissolution *in vitro* d'un co-micronisat selon l'invention. Brièvement, une quantité de co-micronisat représentant 30 mg de principe actif est disposée dans une gélule en gélatine. Cette gélule est ensuite placée dans 900 ml d'un milieu tamponné à pH gastrique, comprenant 0,1% de SDS, à 37±0,5 °C et soumis à une agitation de 50 tours par minute (rpm) (vitesse de rotation des pales du dispositif de dissolution). La dissolution du principe actif dans le milieu peut être suivie par spectrophotométrie à la longueur d'onde maximale d'absorbance. Un pH gastrique est typiquement un pH de 1 à 3.

La présente invention a également pour objet un procédé de préparation du co-micronisat ci-dessus décrit, comprenant les étapes consistant à :
a) fournir un principe actif choisi parmi le groupe constitué par l'ulipristal acétate, un métabolite de l'ulipristal acétate et leurs mélanges,
b) mélanger le principe actif de l'étape a) avec un surfactant acceptable sur le plan pharmaceutique et
c) microniser le mélange obtenu à l'étape b).

Comme cela a été montré dans les exemples, le principe actif fourni à l'étape a) peut être sous forme micronisée ou non-micronisée. Par ailleurs, le principe actif peut être amorphe ou cristallin. De manière préférée, le principe actif fournit à l'étape a) est sous forme cristalline.

Le surfactant mis en oeuvre à l'étape b) peut être non-micronisé ou micronisé.

L'étape c) de micronisation peut être réalisée à l'aide d'un système de micronisation disponible dans le commerce. Il peut s'agir, en particulier, d'un microniseur à jet d'air ou d'un microniseur à boules. L'homme du métier, grâce à ses connaissances générales et à la mise en oeuvre d'expériences de routine, saura déterminer les conditions de mise en oeuvre de l'étape c) afin d'obtenir un produit de co-micronisation présentant la granulométrie désirée. A titre d'exemple, lorsque l'étape c) est mise en oeuvre à l'aide d'un microniseur à jet d'air, l'homme du métier pourra faire varier le débit d'alimentation en poudre et la pression des jets d'air afin de moduler la granulométrie du co-micronisat final.

### Composition pharmaceutique selon l'invention

Le produit de co-micronisation est destiné principalement à une utilisation thérapeutique ou contraceptive. A cette fin, il peut être administré directement ou inséré dans un dispositif d'administration tel qu'un anneau vaginal, un patch, un stérilet ou un implant.

En général, le produit de co-micronisation selon l'invention est intégré dans une composition pharmaceutique de manière à faciliter son administration. Ainsi, un objet supplémentaire de la présente invention est une composition pharmaceutique comprenant un produit de co-micronisation tel que défini précédemment et au moins un excipient acceptable sur le plan pharmaceutique.

L'homme du métier saura choisir le ou les excipients à associer au produit de co-micronisation en fonction de la forme finale de la composition pharmaceutique, de la voie d'administration souhaitée et du profil de libération du principe actif recherché. A cette fin, l'homme du métier pourra se référer aux ouvrages de référence suivants : Remington: The Science and Practice of Pharmacy (Lippincott Williams & Wilkins; Twenty first Edition, 2005) et, Handbook of Pharmaceuticals Excipients, American Pharmaceutical Association (Pharmaceutical Press; 6th revised édition, 2009).

La composition pharmaceutique et le co-micronisat selon l'invention peuvent être administrés par toute voie, en particulier par voie orale, buccale, nasale, sublinguale, vaginale, intrautérine, rectale, transdermale ou par voie parentérale, par exemple par injection intraveineuse. Les voies d'administration préférées sont les voies buccale, orale, intrautérine et vaginale.

La composition pharmaceutique selon l'invention peut se présenter sous toute forme, par exemple sous la forme d'un comprimé, d'une poudre, d'une gélule, d'une pilule, d'un suppositoire, d'une ovule, d'une suspension, d'une solution aqueuse, alcoolique ou huileuse, d'un sirop, d'un gel, d'une pommade, d'une émulsion, d'un lyophilisat ou d'un film orodispersible. La voie d'administration et la forme galénique de la composition pharmaceutique pourront dépendre de l'effet thérapeutique ou contraceptif recherché.

Dans certains modes de réalisation, la composition pharmaceutique selon l'invention peut être intégrée dans un dispositif permettant l'administration prolongée du principe actif. La composition pharmaceutique pourra être, en particulier, intégrée dans un anneau vaginal, dans un stérilet, dans un patch, par exemple un patch transdermique ou muco-adhésif, ou dans un implant, par exemple un implant de type contraceptif. Pour des exemples d'anneaux vaginaux adaptés à la mise en oeuvre de l'invention, on pourra se référer à la demande WO2006/10097.

Dans des modes de réalisation supplémentaires, la composition pharmaceutique selon l'invention se présente sous forme solide. De préférence, la composition pharmaceutique selon l'invention est solide et destinée à une administration orale.

Dans certains modes de réalisation, la composition pharmaceutique selon l'invention est caractérisée en ce que l'excipient acceptable sur le plan pharmaceutique est choisi parmi le groupe constitué par un diluant, un liant, un agent d'écoulement, un lubrifiant, un agent désintégrant et leurs mélanges.

Un diluant au sens de la présente invention peut être un ou plusieurs composés capables de densifier le principe actif pour obtenir la masse désirée. Les diluants englobent les phosphates minéraux, les monosaccharides et les polyols tels que le xylitol, le sorbitol, le lactose, le galactose, le xylose ou le mannitol, les disaccharides tels que le saccharose, les oligosaccharides, les polysaccarides tels que la cellulose et ses dérivés, les amidons, et les mélanges. Le diluant peut être sous forme anhydre ou hydraté. A titre d'exemple, un diluant adapté peut être choisi parmi la cellulose microcristalline, le mannitol, le lactose et leurs mélanges.

Le liant peut être un ou plusieurs composés capables d'améliorer l'agrégation du principe actif avec le diluant. On peut citer, à titre d'exemple de liants, l'hydroxypropyl cellulose, l'hydroxypropylméthyl cellulose, la povidone (polyvinylpyrrolidone), les copolymères de N-vinyl-2-pyrrolidone et d'acétate de vinyle (copovidone) et leurs mélanges.

Le lubrifiant peut être un ou plusieurs composés capables d'empêcher les problèmes liés à la préparation des formes galéniques sèches, comme les problèmes de collage et/ou de grippage qui surviennent au niveau des machines lors de la compression ou du remplissage. Les lubrifiants préférés sont des acides gras ou des dérivés d'acides gras comme le stéarate de calcium, le monostéarate de glycéryle, le palmitostéarate de glycéryle, le stéarate de magnésium, le stéarate de zinc, ou l'acide stéarique, les polyalkylèneglycols, notamment le polyéthylèneglycol, le benzoate de sodium ou le talc. Les lubrifiants préférés selon l'invention sont les sels de stéarates et leurs mélanges. Un lubrifiant adapté est par exemple le stéarate de magnésium.

L'agent d'écoulement éventuellement employé selon invention peut être choisi parmi les composés qui contiennent du silicium, par exemple, le talc, la silice colloïdale anhydre ou de la silice précipitée.

Le désintégrant peut être utilisé pour améliorer la libération du principe actif. Il peut être choisi, par exemple, parmi la polyvinyl pyrrolidone réticulée (crospovidone), la carboxyméthyl cellulose réticulée (telle que la croscarmellose de sodium) ou non-réticulée, les amidons et leurs mélanges. Le désintégrant est de préférence choisi dans le groupe constitué par une croscarmellose sodique, une crospovidone et leurs mélanges.

Dans certains modes de réalisation, la composition selon l'invention comprend :
- 0,5% à 80% du produit de co-micronisation tel que défini précédemment,
- 0% à 10% d'agent désintégrant,
- 15% à 95 % de diluant, et
- 0% à 5% de lubrifiant,
les pourcentages étant exprimés en poids par rapport au poids total de la composition.

La composition selon l'invention peut être, en outre, caractérisée en ce qu'elle comprend de 0% à 20% en poids d'un agent liant, et de 0% à 5% en poids d'un agent d'écoulement.

Dans d'autres modes de réalisation, la composition selon l'invention comprend:
- 1% à 65% du produit de co-micronisation tel que défini précédemment,
- 0% à 10% d'agent désintégrant, de préférence choisi parmi une croscarmellose sodique, une crospovidone et leurs mélanges,
- 25% à 95 % de diluant, de préférence choisi parmi le mannitol, le lactose, la cellulose microcristalline et leurs mélanges et
- 0% à 5% de lubrifiant de préférence un stéarate tel que le stéarate de magnésium,
les pourcentages étant exprimés en poids par rapport au poids total de la composition.

Dans un mode réalisation supplémentaire, la composition selon l'invention comprend
- 1% à 45% du produit de co-micronisation,
- 0% à 10% d'agent désintégrant, de préférence choisi parmi une croscarmellose sodique, une crospovidone et leurs mélanges,
- 40% à 95 % de diluant, de préférence choisi parmi le mannitol, le lactose, la cellulose microcristalline et leurs mélanges et
- 0% à 3% de lubrifiant, de préférence un stéarate tel que le stéarate de magnésium,

A titre d'exemple, la composition pharmaceutique selon l'invention peut comprendre de :
- 1% à 10% en poids de produit de co-micronisation,
- 80% à 95% en poids de diluant,
- 1% à 8% en poids d'agent désintégrant, et
- 0,1 % à 2% d'un lubrifiant,
les pourcentages étant exprimés en poids par rapport au poids total de la composition.

Un exemple supplémentaire est une composition pharmaceutique comprenant de :
- 35% à 45% en poids de produit de co-micronisation,
- 50% à 60% en poids de diluant,
- 1% à 8% en poids d'agent désintégrant, et
- 0,1 % à 2% d'un lubrifiant,
les pourcentages étant exprimés en poids par rapport au poids total de la composition. Il va de soi que, dans les exemples ci-dessus décrits, le produit de co-micronisation comprend, de préférence, de l'ulipristal acétate en tant que principe actif du dodécylsulfate de sodium en tant que surfactant selon un rapport massique de 0,8 à 2,5. La composition pharmaceutique selon l'invention peut comprendre, en outre, un ou plusieurs excipients additionnels aux excipients précédemment cités. Le ou les excipients additionnels peuvent être choisis parmi le groupe constitué par les agents d'enrobage tels que les agents d'enrobage à base d'alcool polyvynilique ou d'hydroxypropylmethylcellulose, les pigments tels que l'oxyde d'aluminium ou l'oxyde de fer, les arômes, les agents mouillants, les cires, les agents dispersants, les stabilisants et les conservateurs.

Dans certains modes de réalisation, la composition pharmaceutique selon l'invention est dépourvue d'agent liant et/ou d'agent d'écoulement. Dans d'autres modes de réalisation, le SDS présent dans le produit de co-micronisation est le seul surfactant présent dans la composition pharmaceutique.

La composition pharmaceutique selon l'invention pourra être préparée selon l'une quelconque des méthodes couramment utilisées en galénique. Ces méthodes comprennent typiquement le mélange du produit de co-micronisation selon l'invention avec un ou plusieurs excipients puis la mise en forme du mélange obtenu. A titre d'exemple, lorsqu'elle se présente sous la forme d'un comprimé, la composition pharmaceutique selon l'invention peut être préparée, par compression directe ou par compression après granulation par voie sèche ou humide.

Dans les modes de réalisation ci-dessus décrits de la composition pharmaceutique selon l'invention, le produit de co-micronisation est, de préférence, caractérisé en ce qu'il comprend de l'UPA et du SDS, le rapport massique UPA/SDS étant de 0,5 à 4, de préférence de 0,8 à 2,5. Il va de soi que le produit de co-micronisation intégré dans la composition pharmaceutique selon l'invention peut avoir l'une quelconque des caractéristiques décrites dans la présente description. En particulier, le produit de co-micronisation présente une ou plusieurs (1, 2, 3, 4 ou 5) des caractéristiques suivantes :
i. le principe actif est l'UPA et le surfactant est le SDS,
ii. le rapport massique principe actif/surfactant est de 0,8 à 1,2,
iii. la d50 du produit de co-micronisation est inférieure à 20 µm, de préférence inférieure à 15 µm,
iv. la d90 du produit de comicronisation est inférieure à 50 µm, de préférence inférieure à 40 µm, et
v. au moins 80% du principe actif que le produit de co-micronisation contient est libéré en 30 minutes lorsque ledit produit de co-micronisation est soumis à un test de dissolution *in vitro,* de préférence dans les conditions suivantes :
   - dispositif: dispositif de dissolution à pales,
   - échantillon : gélule en gélatine contenant une quantité de co-micronisat correspondant à 30 mg de principe actif,
   - milieu de dissolution : 900 ml d'une solution aqueuse tamponnée à pH gastrique comprenant 0,1% de SDS,
   - température : 37±0,5 °C, et
   - vitesse de rotation des pales : 50 tours par minute (rpm)

Un autre exemple supplémentaire selon l'invention est une composition pharmaceutique comprenant de :
- 4% à 10% en poids de produit de co-micronisation selon l'invention, de préférence comprenant de l'UPA et du SDS selon un rapport massique UPA/SDS de 0,8 à 2,5, de préférence de 0,8 à 1,2,
- 50% à 65% en poids de cellulose microcristalline et de 25% à 35 % en poids de mannitol en tant que diluants,
- 1% à 8% en poids de crospovidone et/ou de croscarmellose de sodium en tant que désintégrant, et
- 0,1% à 2% de stéarate de magnésium en tant que lubrifiant.

Comme mentionné précédemment, la composition selon l'invention peut se présenter sous la forme d'une poudre, d'une granule, d'un comprimé pelliculé ou non pelliculé, ou d'une gélule, et est de préférence destinée à une administration orale. Dans certains modes de réalisation, la composition pharmaceutique selon l'invention se présente sous la forme d'un comprimé non-pelliculé destiné à une administration orale.

La composition selon l'invention peut être une composition pharmaceutique à libération contrôlée, immédiate, prolongée ou retardée. De préférence, la composition selon l'invention est une composition à libération immédiate.

On entend par une « composition à libération immédiate », une composition pharmaceutique caractérisée en ce qu'au moins 75% du principe actif initialement contenu dans une unité de dose de la composition pharmaceutique est libéré en 45 minutes lorsque ladite unité de dose est soumise à un test de dissolution in vitro, par exemple tel que défini dans la Pharmacopée Européenne §2.9.3, et de préférence, dans les conditions suivantes :
- dispositif de dissolution à pales,
- milieu de dissolution : solution aqueuse tamponnée à pH gastrique contenant 0,1% de SDS
- température : 37±0.5°C, et
- vitesse de rotation : 50 rpm.

Le volume du milieu de dissolution dépend de la quantité de principe actif contenue dans l'unité de dose. Pour une unité de dose comprenant 5 mg de principe actif, on utilise 500 ml de milieu de dissolution. Pour une unité de dose comprenant 30 mg de principe actif, on utilise 900 ml de milieu de dissolution.

Dans certains modes de réalisation, la composition selon l'invention est caractérisée en ce qu'au moins 60%, voire au moins 70% du principe actif présent dans une unité de dose est libéré en 20 minutes, lorsque ladite unité de dose est soumise à un test de dissolution in vitro réalisé de préférence dans les conditions décrites ci-dessus.

« Au moins 70% du principe actif présent dans une unité de dose » englobe au moins 72%, au moins 74%, au moins 76%, au moins 78%, au moins 80%, au moins 82%, au moins 86% du principe actif présent dans une unité de dose.

Dans certains modes de réalisation, la composition selon l'invention est caractérisée en ce qu'au moins 80% du principe actif présent dans une unité de dose est libéré en 20 minutes, lorsque ladite unité de dose est soumise à un test de dissolution in vitro réalisé de préférence dans les conditions décrites ci-dessus.

De manière générale, la composition pharmaceutique comprend de 1 mg à 100 mg de principe actif par unité de dose, de préférence de 1 mg à 40 mg, voire de 2 mg à 30 mg, de principe actif par unité de dose. La dose de principe actif est fonction de l'effet thérapeutique ou contraceptif et du schéma d'administration recherchés. Par exemple pour certaines applications, la quantité d'UPA par unité de dose peut être comprise dans une gamme allant de 1 mg à 5 mg.

En contraception d'urgence, le principe actif peut être présent à hauteur de 20 mg à 40 mg par unité de dose.

En contraception régulière, le principe actif peut être présent à hauteur de 2 mg à 5 mg par unité de dose.

Pour des utilisations thérapeutiques tels que le traitement des fibromes utérins, le principe actif peut être présent à hauteur de 3 mg à 15 mg par unité de dose.

La dose en principe actif et le schéma d'administration pourra également dépendre des paramètres personnels du patient, en particulier de son poids, son âge, son sexe, son état de santé général, son régime alimentaire, des pathologies dont il souffre...

Enfin, la composition pharmaceutique selon l'invention peut comprendre un principe actif supplémentaire. Cet actif supplémentaire peut exercer une action distincte de celle de l'UPA ou de ses métabolites. Il peut également renforcer l'effet thérapeutique de l'UPA ou de ses métabolites.

### Utilisations thérapeutique ou contraceptiques du co-micronisat et de la composition pharmaceutique selon la présente invention

Dans un aspect supplémentaire, la présente invention a également pour objet un produit de co-micronisation ou une composition pharmaceutique tels que décrits précédemment pour une utilisation en tant que médicament. Le produit de co-micronisation ou la composition selon l'invention sont particulièrement adaptés pour une utilisation en tant que contraceptif régulier ou contraceptif d'urgence. Ils peuvent être également utilisés pour le traitement ou la prévention de troubles hormonaux, gynécologiques ou endocriniens tels que la maladie de Cushing. La composition ou le produit de co-micronisation selon l'invention peuvent être utilisés, en particulier, dans le traitement ou la prévention d'un trouble gynécologique, de préférence affectant l'utérus, y compris les troubles gynécologiques bénins. Les troubles gynécologiques englobent, sans y être limités, les fibromes utérins et ses symptômes, l'adénomyose, l'endométriose, les douleurs associées à la dislocation de l'endomètre, les saignements utérins excessifs. Un objet supplémentaire de l'invention est l'utilisation du produit de co-micronisation selon l'invention pour la préparation d'un contraceptif ou pour la préparation d'un médicament destiné au traitement ou à la prévention de l'une quelconque des pathologies ci-dessus citées.

L'invention a également pour objet une méthode de contraception comprenant l'administration à une patiente d'une dose contraceptive du produit de co-micronisation ou de la composition pharmaceutique selon l'invention.

On entend par « méthode de contraception » une méthode permettant d'éviter la survenue d'une grossesse chez une patiente en âge de procréer.

Dans le cas d'espèce, il peut s'agir d'une méthode de contraception d'urgence. Dans ce cas, une dose unique est de préférence administrée à la patiente dans un intervalle de temps adéquate après le rapport non ou mal protégé, en général dans les 120 h suivant le rapport non ou mal protégé.

La méthode de contraception peut être également une méthode de contraception régulière, dans laquelle la composition ou le produit de co-micronisation sont administrés de manière chronique et cyclique à la patiente ou de manière continue à l'aide d'un dispositif tel qu'un implant ou un anneau vaginal.

A titre alternatif, la méthode de contraception peut être une méthode de contraception dite « à la demande » telle que décrite dans la demande internationale WO2010/119029. Enfin, l'invention a également pour objet une méthode de traitement d'une maladie ou d'un trouble chez un patient comprenant l'administration d'une dose thérapeutiquement efficace du produit de co-micronisation ou de la composition pharmaceutique selon l'invention à un patient, de préférence de sexe féminin. La méthode thérapeutique selon l'invention concerne, de préférence, l'un quelconque des maladies ou troubles cités ci-dessus.

Il va de soi que pour la mise en oeuvre des méthodes et utilisations ci-dessus décrites, le produit de co-micronisation et la composition pharmaceutique selon l'invention peuvent comprendre une ou plusieurs des caractéristiques détaillées dans la présente description.

Les exemples ci-après ont pour but d'illustrer plus pleinement l'invention sans pour autant en limiter la portée.

### EXEMPLES

### Exemple 1 : Criblage d'excipients pour la co-micronisation de l'ulipristal acétate (UPA)

### 1. Matériel et méthode

### ▪ Préparation des co-micronisats

Les produits de co-micronisation (ci-après « co-micronisats ») d'ulipristal acétate ont été préparés selon la méthode suivante : L'ulipristal acétate et l'excipient de co-micronisation à tester ont été mélangés selon le rapport massique désiré dans un mortier et triturés jusqu'à l'obtention d'un mélange homogène. Le mélange obtenu a été ensuite micronisé dans un broyeur-homogénéiseur à billes.

### ▪ Dissolution in vitro des co-micronisats d'UPA

Pour chaque co-micronisat obtenu, on a préparé des gélules en gélatine dure contenant une quantité de co-micronisat correspondant à 30 mg d'UPA par gélule. Les études de dissolution *in vitro* de l'UPA en co-micronisat ont été réalisées à partir de ces gélules selon la la Pharmacopée Européenne au §2.9.3 en utilisant un dispositif de dissolution à pales.

Pour chaque co-micronisat, une gélule contenant ledit co-micronisat a été placée dans un bol du dispositif de dissolution contenant 900 ml d'un milieu de dissolution. Le milieu de dissolution est une solution aqueuse tamponnée à pH gastrique et comprenant 0,1% en poids de SDS. Les conditions de mise en oeuvre des dissolutions *in vitro* sont les suivantes :
▪ Vitesse de rotation des pales : 50 tours par minute (rpm)
▪ Température : 37°C ± 0,5°C

La dissolution de l'UPA a été suivie par spectrophotométrie.

A titre d'expérience témoin, on a utilisé une gélule en gélatine contenant 30 mg d'ulipristal acétate micronisé seul (c'est-à-dire d'UPA micronisé en l'absence de tout excipient de co-micronisation).

Pour chaque co-micronisat, l'expérience de dissolution a été reproduite 3 fois.

### 2. Résultats

### ▪ Criblage d'excipients de co-micronisation

Le tableau 1 ci-dessous et la Figure 1 montrent les résultats de dissolution obtenus pour chaque co-micronisat préparé. Les pourcentages de dissolution sont exprimés par rapport à la quantité initiale d'UPA contenue dans chaque gélule.

**Tableau 1 : Résultats des essais de dissolution in vitro pour les co-micromisats d'UPA/excipient préparés. Rapport massique UPA/excipient 7/3. Expérience témoin : UPA micronisé seul.**

| | Pourcentages d'UPA libéré exprimés par rapport à la quantité initiale d'UPA contenue par gélule. (Valeurs moyennes sur 3 expériences) | | | | |
|---|---|---|---|---|---|
| Temps (min) | UPA micronisé seul | UPA/SDS | UPA/Kollicoat® IR | UPA/acide citrique monohydrate | UPA/acide fumarique |
| 0 | 0,0 | 0,0 | 0,0 | 0,0 | 0,0 |
| 1 | 0,0 | 0,0 | 0,2 | 0,1 | 1,1 |
| 5 | 5,5 | 5,3 | 0,5 | 0,5 | 1,2 |
| 7,5 | 15,00 | 15,8 | 0,7 | 0,6 | 1,8 |
| 10 | 22,30 | 31,9 | 0,9 | 0,8 | 2,1 |
| 15 | 30,00 | 67,3 | 1,1 | 1,1 | 2,3 |
| 20 | 34,30 | 80,5 | 1,4 | 1,6 | 2,9 |
| 30 | 39,50 | 85,8 | 2,5 | 2,6 | 5,7 |
| 45 | 47,50 | 89,3 | 5,3 | 4,7 | 11,0 |
| 60 | 53,80 | 91,5 | 8,8 | 7,1 | 16,4 |

On précise que le Kollicoat IR® est un copolymère greffé de polyéthylène glycol et d'alcool polyvinylique.

Ces résultats montrent que la co-micronisation de l'ulipristal acétate avec le dodécyl sulfate de sodium (SDS) permet d'améliorer de manière très significative la vitesse de dissolution et la quantité finale libérée d'UPA. De manière remarquable, le pourcentage d'ulipristal acétate libérée dans le milieu de dissolution à t = 20 min est d'environ 80% pour une gélule comprenant le co-micronisat UPA/SDS alors qu'il n'est que d'environ 35% pour une gélule contenant de l'ulipristal acétate qui a été micronisé en l'absence d'excipient.

Le co-micronisat d'UPA avec le Kollicoat IR® présente une vitesse de libération de l'UPA très inférieure à celle observée pour l'UPA micronisé puisqu'au bout de 60 min, moins de 10% de l'UPA contenu initialement dans les co-micronisats est libéré.

Comme cela est illustré dans le tableau 2 ci-dessous, la solubilité de l'ulipristal acétate diminue de manière très significative en fonction du pH du milieu. Il était donc attendu que la co-micronisation de l'ulipristal d'acétate avec un excipient acide - tel que l'acide citrique ou l'acide fumarique - permette d'améliorer la dissolution de l'ulipristal acétate en diminuant le pH dans l'environnement proche de la forme de dosage et donc en augmentant localement sa solubilité.

**Tableau 2 : solubilité de l'UPA en fonction du pH**

| pH | Solubilité UPA (g/L) |
|---|---|
| 1,2 | 22,7 |
| 4,5 | 0,039 |
| 6,8 | 0,005 |

De manière surprenante, contrairement à ce que l'on aurait pu s'attendre au regard de la solubilité de l'UPA en milieu acide, la co-micronisation de l'UPA avec un acide organique entraîne une diminution nette de la vitesse de dissolution de l'UPA.

### Conclusion

La co-micronisation en présence de SDS a permis d'augmenter de manière significative la vitesse de dissolution et le taux de libération de l'UPA *in vitro* par rapport à l'UPA sous forme micronisée. En revanche, contrairement à ce que l'on aurait pu attendre, les autres excipients de co-micronisation testés dans l'exemple 1 ont eu un impact nettement négatif sur la libération de l'UPA. L'augmentation de la vitesse de dissolution est donc un effet spécifique du SDS.

### ▪ Influence du rapport massique UPA/SDS sur la dissolution in vitro de l'UPA

Différents co-micronisats UPA/SDS ont été préparés selon la méthode de co-micronisation ci-dessus décrite afin d'étudier l'influence du rapport massique UPA/SDS sur la vitesse de dissolution *in vitro* de l'UPA. A titre de comparaison, un co-micronisat UPA/SDS/acide tartrique selon un rapport massique 6/3/1 a été préparé afin de confirmer l'effet d'un excipient de co-micronisation de type acide organique sur la dissolution de l'UPA.

Les dissolutions *in vitro* obtenues sont illustrées à la Figure 2 et présentées dans le Tableau 3 ci-après.

**Tableau 3 : Dissolution in vitro de l'UPA en fonction du rapport massique UPA/SDS des co-micronisats. Expériences témoins : UPA micronisé seul et co-micronisat UPA/SDS/acide tartrique selon le rapport molaire 6/3/1.**

| | Pourcentages d'UPA libéré exprimés par rapport à la quantité initiale d'UPA contenue par gélule. (Valeurs moyennes sur 3 expériences) | | | | |
|---|---|---|---|---|---|
| Temps (min) | UPA micronisé (en l'absence d'excipient) | UPA/SDS 7/3 | UPA/SDS 6/4 | UPA/SDS 5/5 | UPA/SDS /acide tartrique 6/3/1 |
| 0,0 | 0,0 | 0,0 | 0,0 | 0,0 | 0,0 |
| 1,0 | 0.0 | 0,0 | 0,2 | | 0,0 |
| 5,0 | 5.5 | 5,3 | 33,4 | 14,2 | 3,4 |
| 7,5 | 15.00 | 15,8 | 52,2 | 21,1 | 5,0 |
| 10,0 | 22.30 | 31,9 | 67,4 | 44,0 | 8,3 |
| 15,0 | 30.00 | 67,3 | 75,2 | 73,2 | 16,8 |
| 20,0 | 34.30 | 80,5 | 78,8 | 90,7 | 21,7 |
| 30,0 | 39.50 | 85,8 | 82,3 | 99,0 | 30,6 |
| 45,0 | 47.50 | 89,3 | 92,3 | 102,9 | 47,4 |
| 60,0 | 53.80 | 91,5 | 94,5 | 104,4 | 53,9 |

### Conclusion

La vitesse de dissolution de l'UPA augmente avec la diminution du rapport massique UPA/SDS. La présence d'une faible proportion d'acide tartrique dans le co-micronisat a un impact négatif sur la vitesse de dissolution de l'UPA qui n'est pas totalement compensé par la présence du SDS. Ceci confirme les résultats obtenus précédemment avec l'acide fumarique et l'acide citrique.

### ▪ Autres exemples de co-micronisat

Un co-micronisat UPA/SDS/crospovidone est préparé par co-micronisation d'un mélange intime de SDS, d'UPA et de crospovidone selon les rapports massiques 5/2/3 à l'aide d'un homogéneiseur-broyeur à billes. Le profil de dissolution *in vitro* est déterminé comme décrit au point 1. ci-avant.

**Tableau 4 : comicronisat UPA/crospovidone/SDS (5/2/3). Pourcentages d'UPA libéré exprimés par rapport à la quantité initiale d'UPA contenue par gélule. (Valeurs moyennes sur 3 expériences).**

| Temps (min) | % d'UPA libéré |
|---|---|
| 0 | 0,0 |
| 1 | 0,4 |
| 5 | 7,1 |
| 7,5 | 18,0 |
| 10 | 28,0 |
| 15 | 58,3 |
| 20 | 67,6 |
| 30 | 80,7 |
| 45 | 85,4 |
| 60 | 86,8 |

### Exemple 2 : Comparaison du profile de dissolution d'un co-micromisat UPA/SDS par rapport à un mélange UPA/SDS

### 1. Matériel et méthode

L'ulipristal acétate et le SDS ont été mélangés dans un rapport massique 1/1 dans un mortier et triturés jusqu'à l'obtention d'un mélange homogène. Une partie du mélange obtenu a été introduit dans des gélules en gélatine dure à raison de 60 mg par gélule (soit 30 mg d'UPA par gélule).

Le mélange restant a été co-micronisé à l'aide d'un microniseur à jet d'air (jet mill Alpine AS 200). Des gélules en gélatine dure ont été remplies avec 60 mg du co-micronisat final (soit une quantité d'UPA de 30 mg/gélule).

La dissolution de l'UPA pour les deux types de gélules a été étudiée dans des conditions et dans un milieu de dissolution identique à ceux de l'exemple 1.

### 2. Résultats

Les dissolutions obtenues pour le co-micronisat UPA/SDS et le mélange UPA/SDS non-micronisé (mélange physique) sont illustrées dans le tableau 5 ci-dessous.

**Tableau 5 : Dissolution in vitro de l'UPA dans le milieu de dissolution de l'Exemple 1 à partir d'un co-micronisat UPA/SDS 1/1 et d'un mélange physique UPA/SDS 1/1 (non co-micronisé).**

| Pourcentages d'UPA libéré exprimés par rapport à la quantité initiale d'UPA contenue dans chaque gélule. (Valeurs moyennes sur 3 expériences) | | |
|---|---|---|
| Temps (min) | Co-micronisat UPA/SDS 1/1 | Mélange physique UPA/SDS 1/1 |
| 0 | 0 | 0 |
| 3 | 29,6 | 32,2 |
| 5 | 69,3 | 61 |
| 7,5 | 86,2 | 68,4 |
| 10 | 94,3 | 71,8 |
| 15 | 96,4 | 76,2 |
| 20 | 96,8 | 79,3 |
| 30 | 97,4 | 83,4 |
| 45 | 97,9 | 86,9 |
| 60 | 98,1 | 88,8 |

Le taux de dissolution de l'UPA est significativement plus élevé pour le co-micronisat que pour le mélange physique après 20 minutes. Ce résultat montre que la co-micronisation a un impact direct sur la dissolution *in vitro* de l'UPA.

### Exemple 3 : Incidence de la source d'UPA et de la granulométrie du co-micronisat sur la vitesse de dissolution de l'UPA.

### 1. Matériel et méthode

### ▪ Préparation des co-micronisats

Les co-micronisats d'ulipristal acétate avec le SDS ont été préparés à l'aide d'un microniseur à jet d'air.

Brièvement, l'ulipristal acétate et le SDS ont été mélangés dans un rapport massique 1/1 dans un mortier et triturés jusqu'à l'obtention d'un mélange homogène.

Le mélange obtenu a été micronisé dans un microniseur (Fluid Energy Loop Mill) selon les conditions suivantes :
▪ Débit d'alimentation : 3 gr/min
▪ Pression Venturi : 40 PSI
▪ Pression de broyage (Mill Pressure) de 10 à 120 PSI en fonction de la granulométrie souhaitée

La pression de broyage a été modulée de manière à modifier la granulométrie du co-micronisat final. La granulométrie - c'est-à-dire la distribution de la taille des particules - des co-micronisats obtenus a été déterminée par diffraction laser (Appareillage : Malvern - Mastersizer 2000SM SCIROCCO 2000, modèle optique : Fraunhofer). Les co-micronisats ont été préparés soit à partir d'ulipristal acétate micronisé, soit à partir d'ulipristal acétate non-micronisé. Le tableau 6 ci-après présente la granulométrie des lots de co-micronisats obtenus en fonction de la source d'UPA et de la pression de broyage.

**Tableau 6: Granulométrie des différents co-micronisats UPA/SDS 1/1 préparés**

| N° lot | Source d'UPA | D10 (µm) | D50 (µm) | D90 (µm) |
|---|---|---|---|---|
| 1 | Non micronisée | 2,54 | 21,35 | 143,89 |
| 2 | | 0,79 | 3,32 | 10,03 |
| 3 | | 0,69 | 2,48 | 6,00 |
| 4 | micronisée | 0,51 | 1,36 | 3,76 |

### ▪ Dissolution in vitro

Pour chaque lot de co-micronisat, on a préparé des gélules en gélatine dure comprenant 60 mg de co-micronisat (soit 30 mg d'UPA par gélule). Les profils de dissolution ont été obtenus dans les conditions décrites dans l'exemple 1.

### 2. Résultats

### ▪ Incidence de la source d'UPA

La Figure 3 montre la courbe de dissolution du co-micronisat d'UPA/SDS obtenue à partir d'UPA non micronisé (lot n°3, Figure 3 : rond plein) et celle du co-micronisat d'UPA obtenu à partir d'une source d'UPA micronisé (lot n°4, Figure 3 : triangle vide). Il est à noter que les deux co-micronisats ont une granulométrie similaire (voir lots n°3 et n°4 - tableau 6). Les deux profils de dissolution montrent que la nature de l'UPA de départ - micronisé ou non-micronisé - n'a pas d'incidence sur les propriétés de dissolution du co-micronisat final.

### ▪ Incidence de la granulométrie du co-micronisat sur la libération de l'UPA

Le tableau 7 ci-dessous et la Figure 3 illustrent les résultats de dissolution *in vitro* obtenus pour les différents lots de co-micronisat.

**Tableau 7 : Résultats de dissolution in vitro obtenus pour les lots de co-micronisats.**

| | Pourcentages d'UPA libéré exprimés par rapport à la quantité initiale d'UPA contenue par gélule. (Valeurs moyennes sur 3 expériences) | | |
|---|---|---|---|
| Temps (min) | Lot n°3 | Lot n°2 | Lot n°1 |
| 0 | 0 | 0 | 0 |
| 3 | 18 | 12,2 | 6,7 |
| 5 | 54,1 | 47,4 | 23,0 |
| 7,5 | 89,7 | 85,0 | 44,0 |
| 10 | 93,9 | 92,9 | 53,9 |
| 15 | 95 | 96,1 | 64,7 |
| 20 | 95,3 | 96,8 | 71,8 |
| 30 | 95,7 | 97,4 | 75,3 |
| 45 | 95,9 | 98,0 | 78,0 |
| 60 | 96 | 98,5 | 79,5 |

Il apparaît que la vitesse de dissolution et le taux final de dissolution de l'UPA pour les lots n°3 (Figure 3, rond plein) et n°2 (Figure 3, carré vide) sont plus élevés que pour le lot n°1(Figure 3, losange plein et). Ceci montre que la granulométrie du co-micronisat peut avoir une incidence sur la vitesse de dissolution de l'UPA.

A toutes fins utiles, on notera que le co-micronisat n°1, en dépit de sa grosse granulométrie, présente une vitesse de dissolution de l'UPA qui est beaucoup plus élevée que celle observée pour l'UPA micronisé seul (c'est-à-dire en l'absence d'excipient de co-micronisation). Ceci confirme, une fois encore, l'effet spécifique de la co-micronisation en présence de SDS sur les propriétés de dissolution *in vitro* de l'UPA.

### Exemple 5 : Etudes pharmacocinétiques chez le rat

Le but de cette étude est d'illustrer que la co-micronisation de l'ulipristal acétate avec le SDS permet d'améliorer le profil de pharmacocinétique, en particulier la biodisponibilité, de l'UPA en comparaison avec l'UPA micronisé.

### 1. Matériel et Méthode

### ▪ Animaux

L'étude de pharmacocinétique a été réaliée sur des rats femelles OFA(SD) dont le poids était compris entre 0,206 et0,251 kg. Les animaux ont été mis au jeun 16 heures avant l'administration des échantillons. Les animaux ont été placés dans des cages dont la température était maintenue entre 20 et 25°C, les cycles jour/nuit étaient de 12 h chacun (6.00 - 18.00) et l'air était conditionné par un système de ventilation.

### ▪ Echantillons d'UPA

Les échantillons testés sont les suivants :
- Co-micronisat ulipristal acétate /SDS ratio 1/1
- Poudre d'ulipristal acétate micronisé en l'absence de tout excipuient (UPA micronisé seul)

### ▪ Protocole d'administration

Une quantité déterminée de poudre a été administrée par tubage oesophagien à l'aide d'une canule. La poudre a été introduite dans l'estomac de chaque animal à l'aide d'un cathéter. La dose administrée correspond à 4 mg d'ulipristal acétate par kg.

### ▪ Prélèvements

Des prises de sang de 300 µl ont été effectuées au niveau de la jugulaire (gauche ou droite) des rats. Pour éviter un prélèvement sanguin trop élevé, les rats ont été divisés en 3 groupes de 6 rats comme suit :

**Tableau 8: Planning de prélèvements**

| Temps de prélèvement | Groupes de rat | | |
|---|---|---|---|
| | N° 1 | N°2 | N°3 |
| Pré administration (Tps = 0) | X | | |
| 10 min | X | | |
| 15 min | | X | |
| 20min | | | X |
| 30 min | X | | |
| 45 min | | | X |
| 1h | | X | |
| 1.25h | X | | |
| 1.5h | | | X |
| 2h | | X | |
| 2.5h | | | X |
| 3h | | X | |
| 4h | | | X |
| 6h | | | X |
| 10h | | X | |
| 16h | X | | |
| 24h | X | | |
| 36h | | X | |

Les échantillons ont été collectés sur tubes à héparine puis centrifugés dans l'heure à 7-8 min 1600g à 4°C. A chaque temps de prélèvement, le plasma des 6 rats du groupe est « poolé »

### ▪ Méthodes de dosage de l'UPA dans les échantillons plasmatiques

L'ulipristal acétate a été dosé par LC/MS/MS dans les conditions suivantes :
- Phase mobile:Méthanol/ eau distillée (70/30, V/V) +1% d'acide acétique
- Colonne:BDS Hypersil phenyl, 100 x 2.1 mm, 5 µm
- Mode d'élution : isocratique
- débit : 0.400 mL/min
- Volume d'injection : 10.0 µL
- Liquide de nettoyage de l'aiguille : Acétonitrile/isopropanol/acétone (40/40/20, V/V/V)

### 2. Résultats

Le tableau 9 ci-dessous présente les résultats de pharmacocinétique obtenus. La co-micronisation permet d'augmenter la biodisponibilité de l'UPA : L'AUC₀₋ₜ et l'AUC_{0-inf} pour le co-micronisat sont d'environ 15% à 20% plus élévés que les AUC obtenus pour l'UPA micronisé. La vitesse d'absorption de l'UPA est également plus rapide avec le co-micronisat (Tₘₐₓ = 1 h) qu'avec l'UPA micronisé (Tₘₐₓ = 1,25 h). De manière remarquable, la co-micronisation n'augmente pas significativement la Cₘₐₓ (variation de +3,6% seulement). Ces résultats *in vivo* sont cohérents avec les résultats de dissolution *in vitro* obtenus précédemment.

**Tableau 9 : Résultats de pharmacocinétique**

| | Co-micronisat UPA/SDS 1/1 | UPA micronisé | Variations |
|---|---|---|---|
| **Cmax (ng/mL)** | 463 | 447 | + 3,6% |
| **Tmax (h)** | 1,0 | 1,25 | - 20% |
| **AUC0-t** (**h**.**ng**/**mL**) | 1595 | 1340 | **+** 19% |
| **AUCinf** (**h**.**ng**/**mL**) | 1610 | 1383 | +16,4% |

En conclusion, la co-micronisation en présence de SDS permet d'améliorer de manière significative la biodisponibilité de l'UPA, sans toutefois augmenter de manière drastique la Cmax.

La co-micronisation de l'UPA en présence de SDS permet l'obtention d'une nouvelle matrice de principe actif présentant une biodisponibilité améliorée, qui devrait permettre de diminuer les doses d'UPA à administrer au patient pour atteindre les effets thérapeutiques recherchés._Cette nouvelle matrice devrait permettre également de développer de nouveaux schémas d'administration de l'UPA.

### Exemple 6 : Compositions pharmaceutiques intégrant le co-micronisat selon l'invention

### 1. Matériel et méthode

Afin de confirmer le gain de dissolution de l'UPA, même après formulation du co-micronisat, des comprimés dosés à 5 mg en ulipristal acétate (non-pelliculés) ont été fabriqués par compression directe et testés dans les mêmes conditions de dissolution *in vitro* que celles décrites dans l'exemple 1 à l'exception du volume du milieu de dissolution qui est ici de 500 ml. La composition des comprimés est présentée dans le tableau 10 ci-dessous.

**Tableau 10 : composition des comprimés préparés**

| | **Comprimé n°1 (invention)** | | **Comprimé n°2 (invention)** | |
|---|---|---|---|---|
| Ingrédients | mg/comprimé | % massique | mg/comprimé | % massique |
| UPA/SDS 1/1 | 10,00 | 6,7 | 10,00 | 6,7 |
| Microcristalline cellulose | 92,45 | 61,6 | 87,50 | 58,3 |
| Mannitol | 43,50 | 29,0 | 43,50 | 29,0 |
| Sodium croscarmellose | 2,55 | 1,7 | 7,50 | 5,0 |
| Magnesium stearate | 1,50 | 1,0 | 1,50 | 1,0 |
| Total | 150,00 | 100,0 | 150,00 | 100,0 |

### 2. Résultats

Les profils de dissolution *in vitro* sont illustrés à la Figure 4 et dans le tableau 11 ci-dessous.

**Tableau 11 : Résultats des dissolutions in vitro pour les comprimés n°1 et n°2**

| | Pourcentages de dissolution de l'UPA | |
|---|---|---|
| Temps (min) | Comprimé n° 1 (Invention) | Comprimé n°2 (invention) |
| 0 | 0 | 0 |
| 3 | 17,3 | 20,6 |
| 5 | 31,4 | 43,1 |
| 7,5 | 48,2 | 65,5 |
| 10 | 60,7 | 81,7 |
| 15 | 70,2 | 85,9 |
| 20 | 74,2 | 87,3 |
| 30 | 78,2 | 88,6 |
| 45 | 82,2 | 89,9 |
| 60 | 84,7 | 90,9 |

Les comprimés selon l'invention présentent des vitesses et un taux final de libération de l'UPA élevés. Ces résultats confirment que la co-micronisation de l'UPA avec le SDS permet d'améliorer ses propriétés de solubilisation, même après intégration dans une composition pharmaceutique complexe.

Enfin, au cours de la préparation des comprimés, le Demandeur a observé que le produit de co-micronisat était beaucoup plus facile à formuler que l'UPA micronisé sans SDS. En effet, le Demandeur a noté que la formulation utilisée pour la préparation des comprimés n°1 et n°2 était fluide et facilement compressible même en l'absence de talc (agent d'écoulement) et de liant. En d'autres termes, le co-micronisat selon l'invention peut être facilement formulé même en l'absence d'agent d'écoulement.

### 3. Tests comparatifs

3 lots supplémentaires de comprimés ont été préparés par compression directe. Le lot de comprimés n°3 correspond à l'invention. Les lots n°4 et n°5 correspondent à des lots comparatifs puisqu'ils sont dépourvus de co-micronisat. Les comprimés n°4 comprennent de l'UPA micronisé et du SDS alors que les comprimés n°5 comprennent de l'UPA micronisé seulement.

**Tableau 12 : Exemples de comprimés**

| | Comprimé n°3 (invention) | Comprimé n°4 (comparatif) | Comprimé n°5 (comparatif) |
|---|---|---|---|
| Ingrédients | mg/comprimé | mg/comprimé | mg/comprimé |
| UPA micronisé | 0 | 5 | 5 |
| SDS | 0 | 5 | 0 |
| Co-micronisat UPA/SDS 1/1 | 10 | 0 | 0 |
| Cellulose microcristalline | 88,25 | 88,25 | 93,25 |
| Mannitol | 43,5 | 43,5 | 43,5 |
| Crospovidone | 7,5 | 7,5 | 7,5 |
| Stéarate de magnésium | 0,75 | 0,75 | 0,75 |
| TOTAL | 150 | 150 | 150 |

Les profils de dissolution *in vitro* pour ces comprimés ont été obtenus selon les conditions décrites au point 1. ci-avant et sont illustrés dans le tableau 13 ci-dessous.

**Tableau 13 : Résultats des dissolutions in vitro pour les comprimés n°3 (invention), n°4 (comparatif) et n°5 (comparatif).**

| | Pourcentages de dissolution de l'UPA | | |
|---|---|---|---|
| Time (min) | Comprimé n°3 (invention) | Comprimé n°4 (comparatif) | Comprimé n°5 (comparatif) |
| 0 | 0 | 0 | 0 |
| 3 | 6,8 | 12,6 | 26,7 |
| 5 | 54,3 | 56,2 | 43 |
| 7,5 | 79,2 | 60,5 | 46 |
| 10 | 87,2 | 62,6 | 47,8 |
| 15 | 88,9 | 65,4 | 49,6 |
| 20 | 89,8 | 67,5 | 50,9 |
| 30 | 90,9 | 70,7 | 52,7 |
| 45 | 92,1 | 74,7 | 55,3 |
| 60 | 93,1 | 78,3 | 57,3 |

Il apparaît que la vitesse et le taux final de dissolution de l'UPA pour le comprimé selon l'invention (comprimé n°3) sont plus élevés que ceux du comprimé n°4 (mélange de SDS et d'UPA micronisé) et du comprimé n°5 (UPA micronisé).

Ces résultats confirment l'effet spécifique de la co-micronisation en présence d'un surfactant tel que le SDS sur les propriétés de solubilisation de l'UPA, même après incorporation dans une composition pharmaceutique.

A toutes fins utiles, on précise que les compositions selon l'invention illustrées dans les tableaux 10 et 12 peuvent être utilisées, par exemple, en contraception régulière ou à la demande, ou encore en tant que médicament, par exemple pour le traitement de troubles gynécologiques tels que le fibrome utérin.

### Exemple 7 : Exemples supplémentaires de compositions selon l'invention

Le tableau 14 ci-après présente un exemple supplémentaire d'une composition selon l'invention. Cette composition peut être préparée et mise en forme par compression directe.

**Tableau 14 : exemple supplémentaire d'une composition selon l'invention**

| **Composition** | **mg/comprimé** | **% massique** |
|---|---|---|
| Co-micronisat UPA/SDS 1/1 | 60 (dont 30 mg d'UPA) | 40 |
| Cellulose microcristalline | 38,25 | 25,5 |
| Mannitol | 43,5 | 29 |
| Crospovidone | 7,5 | 5 |
| Magnésium stéarate | 0,75 | 0,5 |
| Total | 150 | 100 |

Cette composition peut être utilisée en tant que contraceptif d'urgence.

D'autres exemples de comprimés sont fournis ci-après. Ces comprimés peuvent être préparés par compression directe.

| **Composition** | **mg/comprimé** | **% massique** |
|---|---|---|
| Co-micronisat UPA/SDS 1/1 | 60,0 (dont 30 mg d'UPA) | 50 |
| Lactose | 49,2 | 41 |
| Crospovidone | 9,6 | 8 |
| Stéarate de magnésium | 1,2 | 1 |
| Total | 120,0 | 100 |

| **Composition** | **mg/comprimé** | **% massique** |
|---|---|---|
| Co-micronisat UPA/SDS 1/1 | 8,00 (dont 4 mg d'UPA) | 12,5 |
| Lactose | 28,80 | 45 |
| Mannitol | 20,48 | 32 |
| Croscarmellose de sodium | 6,40 | 10 |
| Stéarate de magnésium | 0,32 | 0,5 |
| Total | 64,00 | 100 |

| **Composition** | **mg/comprimé** | **% massique** |
|---|---|---|
| Co-micronisat UPA/SDS 1/1 | 20,0 (dont 10 mg d'UPA) | 25 |
| Lactose | 32,0 | 40 |
| Cellulose microcristalline | 22,4 | 28 |
| Crospovidone | 4,8 | 6 |
| Stéarate de magnésium | 0,8 | 1 |
| Total | 80,0 | 100 |

| **Composition** | **mg/comprimé** | **% massique** |
|---|---|---|
| Co-micronisat UPA/SDS 6/4 | 10,00 (soit 6 mg d'UPA) | 6,7 |
| Microcristalline cellulose | 92,45 | 61,6 |
| Mannitol | 43,50 | 29 |
| Croscarmellose de sodium | 2,55 | 1,7 |
| Stearate de magnésium | 1,50 | 1 |
| Total | 150 | 100 |

| **Composition** | **mg/comprimé** | **% massique** |
|---|---|---|
| Co-micronisat UPA/SDS 7/3 | 42,85 (dont 30 mg d'UPA) | 30,6 |
| Cellulose microcristalline | 45,40 | 32,4 |
| Mannitol | 43,5 | 31,1 |
| Crospovidone | 7,5 | 5,4 |
| Magnésium stéarate | 0,75 | 0,5 |
| Total | 140 | 100 |

## Revendications

1. Produit de co-micronisation comprenant :
- un principe actif choisi parmi le groupe constitué par l'ulipristal acétate, un métabolite de l'ulipristal acétate et leurs mélanges, et
- un surfactant solide acceptable sur le plan pharmaceutique choisi parmi les sels d'alkylsulfates en C₈-C₂₀ et leurs mélanges.

2. Produit de co-micronisation selon la revendication 1 **caractérisé en ce que** le rapport massique entre le principe actif et le surfactant est compris dans une gamme allant de 0,1 à 10, de préférence 0,5 à 4.

3. Produit de co-micronisation selon l'une quelconque des revendications 1 à 2 **caractérisé en ce que** le surfactant est le dodécylsulfate de sodium.

4. Produit de co-micronisation selon l'une quelconque des revendications 1 à 3 **caractérisé en ce que** le principe actif est choisi parmi le groupe constitué par l'ulipristal acétate, la 17α-acétoxy-11β-[4-N-méthylamino-phényl)-19-norprégna-4,9-diène-3, 20-dione, la 17α-acétoxy-11β-[4-amino-phényl)-19-norprégna- 4, 9-diène-3,20-dione et leurs mélanges.

5. Produit de co-micronisation selon l'une quelconque des revendications 1 à 4 **caractérisé en ce que** :
- le surfactant est le dodécylsulfate de sodium, et
- le principe actif est l'ulipristal acétate.

6. Produit de co-micronisation selon l'une quelconque des revendications 1 à 5 ayant:
- une d50 inférieure à 20 µm, de préférence inférieure à 15 µm, et/ou
- une d90 inférieure à 50 µm, de préférence inférieure à 40 µm.

7. Procédé de préparation d'un produit de co-micronisation tel que défini dans l'une quelconque des revendications 1 à 6, comprenant les étapes consistant à :
a) fournir un principe actif choisi parmi le groupe constitué par l'ulipristal acétate, un métabolite de l'ulipristal acétate et leurs mélanges,
b) mélanger le principe actif de l'étape a) avec un surfactant solide acceptable sur le plan pharmaceutique choisi parmi les sels d'alkylsulfates en C₈-C₂₀ et leurs mélanges et
c) co-microniser le mélange obtenu à l'étape b).

8. Procédé de préparation d'un produit de co-micronisation selon la revendication 7 **caractérisé en ce qu'**à l'étape a), le principe actif est fourni sous une forme non-micronisée ou une forme micronisée.

9. Composition pharmaceutique comprenant un produit de co-micronisation tel que défini dans l'une des revendications 1 à 6 et un excipient acceptable sur le plan pharmaceutique

10. Composition pharmaceutique selon la revendication 9 **caractérisée en ce que** l'excipient acceptable sur le plan pharmaceutique est choisi parmi le groupe constitué par un diluant, un liant, un agent d'écoulement, un lubrifiant, un agent désintégrant et leurs mélanges.

11. Composition pharmaceutique selon l'une des revendications 9 et 10 comprenant de :
- 0,5% à 80% de produit de co-micronisation,
- 0% à 10% d'agent désintégrant,
- 15% à 95 % de diluant, et
- 0% à 5% de lubrifiant,
les pourcentages étant exprimés en poids par rapport au poids total de la composition.

12. Composition pharmaceutique selon l'une des revendications 9 à 11 **caractérisée en ce qu'**elle comprend de 1 mg à 100 mg, de préférence de 1 mg à 40 mg, de principe actif par unité de dose.

13. Composition pharmaceutique selon l'une des revendications 9 à 12 **caractérisée en ce qu'**elle est adaptée à une administration par voie orale.

14. Composition pharmaceutique selon l'une des revendications 9 à 13 **caractérisée en ce qu'**elle est sous la forme d'une poudre, d'une granule, d'un comprimé pelliculé ou non pelliculé, ou d'une gélule.

15. Produit de co-micronisation selon l'une quelconque des revendications 1 à 6 ou composition pharmaceutique selon l'une des revendications 9 à 14 pour une utilisation en tant que contraceptif.

16. Produit de co-micronisation selon l'une quelconque des revendications 1 à 6 ou composition pharmaceutique selon l'une des revendications 9 à 14 pour une utilisation dans le traitement ou la prévention d'un trouble gynécologique, de préférence affectant l'utérus.

## Patentansprüche

1. Comikronisierungsprodukt, umfassend:
- einen Wirkstoff, ausgewählt aus der Gruppe, bestehend aus Ulipristalacetat, einem Ulipristalacetat-Metaboliten und Gemischen davon, und
- eine pharmazeutisch verträgliche, feste oberflächenaktive Substanz, ausgewählt aus Salzen von C₈-C₂₀-Alkylsulfaten und Gemischen davon.

2. Comikronisierungsprodukt gemäß Anspruch 1, **dadurch gekennzeichnet, dass** das Massenverhältnis zwischen Wirkstoff und oberflächenaktiver Substanz einen Bereich von 0,1 bis 10, vorzugsweise 0,5 bis 4, umfasst.

3. Comikronisierungsprodukt gemäß einem der Ansprüche 1 bis 2, **dadurch gekennzeichnet, dass** die oberflächenaktive Substanz Natriumdodecylsulfat ist.

4. Comikronisierungsprodukt gemäß einem der Ansprüche 1 bis 3, **dadurch gekennzeichnet, dass** der Wirkstoff aus der Gruppe ausgewählt ist, bestehend aus Ulipristalacetat, 17α-Acetoxy-11β-[4-N-methylamino-phenyl]-19-norpregna-4,9-dien-3,20-dion, 17α-Acetoxy-11β-[4-aminophenyl]-19-norpregna-4,9-dien-3,20-dion und Gemischen davon.

5. Comikronisierungsprodukt gemäß einem der Ansprüche 1 bis 4, **dadurch gekennzeichnet, dass**:
- die oberflächenaktive Substanz Natriumdodecylsulfat ist, und
- der Wirkstoff Ulipristalacetat ist.

6. Comikronisierungsprodukt gemäß einem der Ansprüche 1 bis 5 mit:
- einem d50 kleiner als 20 µm, vorzugsweise kleiner als 15 µm, und/oder
- einem d90 kleiner als 50 µm, vorzugsweise kleiner als 40 µm.

7. Verfahren zur Herstellung eines wie in einem der Ansprüche 1 bis 6 definierten Comikronisierungsprodukts, umfassend die Schritte, bestehend aus:
a) Bereitstellen eines Wirkstoffs, ausgewählt aus der Gruppe, bestehend aus Ulipristalacetat, einem Ulipristalacetat-Metaboliten und Gemischen davon,
b) Mischen des Wirkstoffs von Schritt a) mit einer pharmazeutisch verträglichen, festen oberflächenaktiven Substanz, ausgewählt aus Salzen von C₈-C₂₀-Alkylsulfaten und Gemischen davon, und
c) Comikronisieren des in Schritt b) erhaltenen Gemisches.

8. Verfahren zur Herstellung eines Comikronisierungsprodukts gemäß Anspruch 7, **dadurch gekennzeichnet, dass** in Schritt a) der Wirkstoff in nicht-mikronisierter Form oder mikronisierter Form bereitgestellt wird.

9. Pharmazeutische Zusammensetzung, umfassend ein wie in einem der Ansprüche 1 bis 6 definiertes Comikronisierungsprodukt und einen pharmazeutisch verträglichen Arzneimittelträger.

10. Pharmazeutische Zusammensetzung gemäß Anspruch 9, **dadurch gekennzeichnet, dass** der pharmazeutisch verträgliche Arzneimittelträger aus der Gruppe ausgewählt ist, bestehend aus einem Verdünnungsmittel, einem Bindemittel, einem Fließmittel, einem Gleitmittel, einem Sprengmittel und Gemischen davon.

11. Pharmazeutische Zusammensetzung gemäß einem der Ansprüche 9 und 10, umfassend:
- 0,5% bis 80% Comikronisierungsprodukt,
- 0% bis 10% Sprengmittel,
- 15% bis 95% Verdünnungsmittel, und
- 0% bis 5% Gleitmittel,
wobei die prozentualen Anteile als Gewichtsprozente bezogen auf das Gesamtgewicht der Zusammensetzung ausgedrückt sind.

12. Pharmazeutische Zusammensetzung gemäß einem der Ansprüche 9 bis 11, **dadurch gekennzeichnet, dass** sie 1 mg bis 100 mg, vorzugsweise 1 mg bis 40 mg, Wirkstoff pro Dosiseinheit umfasst.

13. Pharmazeutische Zusammensetzung gemäß einem der Ansprüche 9 bis 12, **dadurch gekennzeichnet, dass** sie einer oralen Verabreichung angepasst ist.

14. Pharmazeutische Zusammensetzung gemäß einem der Ansprüche 9 bis 13, **dadurch gekennzeichnet, dass** sie in Form eines Pulvers, eines Granulats, einer filmüberzogenen oder nichtüberzogenen Tablette oder einer Gelatinekapsel vorliegt.

15. Comikronisierungsprodukt gemäß einem der Ansprüche 1 bis 6 oder pharmazeutische Zusammensetzung gemäß einem der Ansprüche 9 bis 14 für eine Verwendung als Kontrazeptivum.

16. Comikronisierungsprodukt gemäß einem der Ansprüche 1 bis 6 oder pharmazeutische Zusammensetzung gemäß einem der Ansprüche 9 bis 14 für eine Verwendung in der Behandlung oder Prävention einer vorzugsweise den Uterus betreffenden gynäkologischen Störung.

## Claims

1. Co-micronization product comprising:
- an active ingredient selected from the group consisting of ulipustal acetate, a metabolite of ulipristal acetate, and mixtures thereof, and
- a pharmaceutically acceptable solid surfactant selected from C₈-C₂₀ alkyl sulfate salts and mixtures thereof.

2. Co-micronization product according to Claim 1, **characterized in that** the weight ratio between the active ingredient and the surfactant is included in a range from 0.1 to 10, preferably 0.5 to 4.

3. Co-micronization product according to any one of Claims 1 to 2, **characterized in that** the surfactant is sodium dodecyl sulphate.

4. Co-micronization product according to any one of Claims 1 to 3, **characterized in that** the active ingredient is selected from the group consisting of ulipustal acetate, 17α-acetoxy-11β-(4-N-methylaminophenyl)-19-noipregna-4,9-diene-3,20-dione, 17α-acetoxy-11β-(4-aminophenyl)-19-noipregna-4,9-diene-3,20-dione and mixtures thereof.

5. Co-micronization product according to any one of Claims 1 to 4, **characterized in that**:
- the surfactant is sodium dodecyl sulphate, and
- the active ingredient is ulipristal acetate.

6. Co-micronization product according to any one of Claims 1 to 5, having
- a d50 of less than 20 µm, preferably less than 15 µm, and/or
- a d90 of less than 50 µm, preferably less than 40 µm.

7. Method for preparing a co-micronization product as defined in any one of Claims 1 to 6, comprising the steps consisting in:
a) providing an active ingredient selected from the group consisting of ulipristal acetate, a metabolite of ulipristal acetate and mixtures thereof,
b) mixing the active ingredient of step a) with a surfactant selected from C₈-C₂₀ alkyl sulfate salts and mixtures thereof, and
c) co-micronizing the mixture obtained in step b).

8. Method for preparing a co-micronization product according to Claim 7, **characterized in that**, in step a), the active ingredient is provided in a non-micronized form or a micronized form.

9. Pharmaceutical composition comprising a co-micronization product as defined in one of Claims 1 to 6 and a pharmaceutically acceptable excipient.

10. Pharmaceutical composition according to Claim 9, **characterized in that** the pharmaceutically acceptable excipient is selected from the group consisting of a diluent, a binder, a flow agent, a lubricant, a disintegrant and mixtures thereof.

11. Pharmaceutical composition according to Claim 9 or 10, comprising from:
- 0.5% to 80% of co-micronization product,
- 0% to 10% of disintegrant,
- 15% to 95% of diluent, and
- 0% to 5% of lubricant,
the percentages being expressed by weight relative to the total weight of the composition.

12. Pharmaceutical composition according to any one of Claims 9 to 11, **characterized in that** it comprises from 1 mg to 100 mg, preferably from 1 mg to 40 mg, of active ingredient per dose unit.

13. Pharmaceutical composition according to any one of Claims 9 to 12, **characterized in that** it is suitable for oral administration.

14. Pharmaceutical composition according to any one of Claims 9 to 13, **characterized in that** it is in the form of a powder, a granule, a coated or uncoated tablet, or a capsule.

15. Co-micronization product according to any one of Claims 1 to 6 or pharmaceutical composition according to any one of Claims 9 to 14, for use as a contraceptive.

16. Co-micronization product according to any one of Claims 1 to 6 or pharmaceutical composition according to any one of Claims 9 to 14, for use in the treatment or prevention of a gynaecological disorder, preferably affecting the uterus.
